# EUROPEAN PATENT APPLICATION

(11) **EP 1 627 592 A1**
(43) Date of publication of application: **22.02.2006**
(21) Application number: 03768225.9
(22) Date of filing: 25.12.2003
(51) Int. Cl.: A61B 1/00

(54) **CAPSULIZED MEDICAL DEVICE**

(30) Priority: 29.05.2003 JP 2003152956
(71) Applicant: Olympus Corporation, Tokyo 151-0072 (JP)
(72) Inventor: UCHIYAMA, Akio, Yokohama-shi, Kanagawa 222-0003 (JP)
(74) Representative: von Hellfeld, Axel
(86) International application number: PCT/JP2003/016692
(87) International publication number: WO 2004/105590

(57) **Abstract**

A capsule medical device inserted into a body cavity has a receiving device such as an antenna and the like, for receiving data from outside the capsule medical device. This capsule medical device also comprises a non-volatile or volatile storage device in which storage data stored therein can be rewritten based on data received by the receiving device, which enables the change or the like of the setup state or operation.

## Description

### Technical Field

The present invention relates to a capsule medical device for performing medical actions by means of a capsule medical device main body inserted into a human body.

### Background Art

Japanese unexamined Patent Application Publication No. 2000-342522 describes a prior art. This prior art relates to an endoscope device of a type that can be placed in a body cavity, wherein a swallowable endoscope and an external device are connected by radio waves, and a bending operation is performed by means of the external device. Moreover, U.S. Patent No. 6402686 and U.S. Patent No. 6402687 disclose a fully swallowable endoscopic system in which a swallowable endoscope and an external device are connected by radio waves.

Furthermore, Japanese Patent No. 3279409 discloses a medical capsule for medical treatment.

Japanese Unexamined Patent Application Publication No. 2000-342522, U.S. Patent No. 6402686, and U.S. Patent No. 6402687 disclose systems wherein a bending operation, or the like, is carried out by means of a radio signal, from a device located externally to the body, as described above, but if it is necessary to maintain the operational state, or to change same, then a signal must be transmitted by the user, from the external device, and hence operability is low.

Furthermore, in Japanese Patent No. 3279409, no description is provided with regard to optimizing the amount of medicine discharged, and the like.

When a capsule medical device is assembled, conventionally, adjustments are made according to the state of each module, and hence adjusted units are assembled.

However, the output of detecting means, such as an image sensor, a force sensor, or the like, and the operation of medicine discharging means, and of tissue or body fluid recovering means, and the like, is liable to be affected by the state of assembly.

Therefore, it is desirable to carry out adjustment after assembly, but in conventional capsule medical devices, no means is provided for carrying out adjustment after assembly.

Furthermore, when an image sensor is provided in a capsule medical device and an image of the interior of a body cavity is obtained, it is not possible to obtain an accurate image in regions where the body passages are narrow, such as the small intestine, oesophagus, and the like, unless the amount of exposure is reduced to a low level, and yet in organs where the body passages are wide, such as the stomach and large intestine, and the like, it is difficult to obtain an accurate image unless the amount of exposure is increased during photographing. In order to obtain images of suitable exposure for all organs, it is necessary to change the photographing conditions constantly, but with the capsule medical device described in the preceding example, this cannot be achieved.

Therefore, the present invention is devised with this point in view, an object thereof being to provide a capsule medical device whereby the settings of the functions provided in the capsule medical device, such as the sensors, can be changed after the capsule device has been assembled.

Moreover, it is an object of the present invention to provide a capsule medical device whereby the functional settings of the capsule medical device upon shipment, and the adjustment thereof, can be varied, by changing the settings.

Furthermore, it is also an object of the present invention to provide a capsule medical device whereby the capsule medical device can be controlled by means of control signals from an external source, in such a manner that it can be operated in more suitable conditions.

### Disclosure of Invention

A capsule medical device inserted into a body cavity, comprising: a receiving device for receiving data from outside the capsule medical device; and a storage device wherein storage data stored therein can be rewritten on the basis of data received by the receiving device. By means of the aforementioned composition, even after assembling a capsule medical device, it is possible readily to set the settings to a suitable state and to change the device to a more suitable operating state, by changing the storage data stored in the storage means, and the like.

### Brief Description of the Drawings

Fig. 1 to Fig. 12B relate to a first embodiment of the present invention, wherein
Fig. 1 is a block diagram showing the overall composition of a capsule medical device according to a first embodiment of the present invention;
Fig. 2 is a cross-sectional view showing the approximate structure of a capsule medical device;
Fig. 3 shows typical data contents stored in a memory or non-volatile memory;
Fig. 4 shows the format used when transmitting information by radio communications;
Fig. 5 shows a typical command;
Fig. 6A and Fig. 6B show illustrative diagrams of a state where the imaging range of the image sensor is determined;
Fig. 7 shows a state where the device is set to a suitable luminance distribution position on a luminance histogram;
Fig. 8 shows a state where the color balance (luminance distribution position for each color) is set to a suitable position;
Fig. 9A shows a composition of an illumination circuit using light-emitting devices;
Fig. 9B shows an illustrative diagram of the operation of the illumination circuit;
Fig. 10 is a circuit diagram showing the composition of a force sensor;
Fig. 11 shows an imaging mode and command code relating to imaging;
Fig. 12A shows a flowchart of processing in the case of a single-frame imaging mode;
Fig. 12B shows a flowchart of processing in the case of a continuous imaging mode;
Fig. 13 is a block diagram showing the overall composition of a capsule medical device according to a second embodiment of the present invention;
Fig. 14 is a block diagram showing the overall composition of a capsule medical device according to a third embodiment of the present invention; and
Fig. 15 is a block diagram showing the overall composition of a capsule medical device according to a fourth embodiment of the present invention.

### Best Mode for Carrying Out the Invention

Below, embodiments of the present invention are described with reference to the drawings.

### (First embodiment)

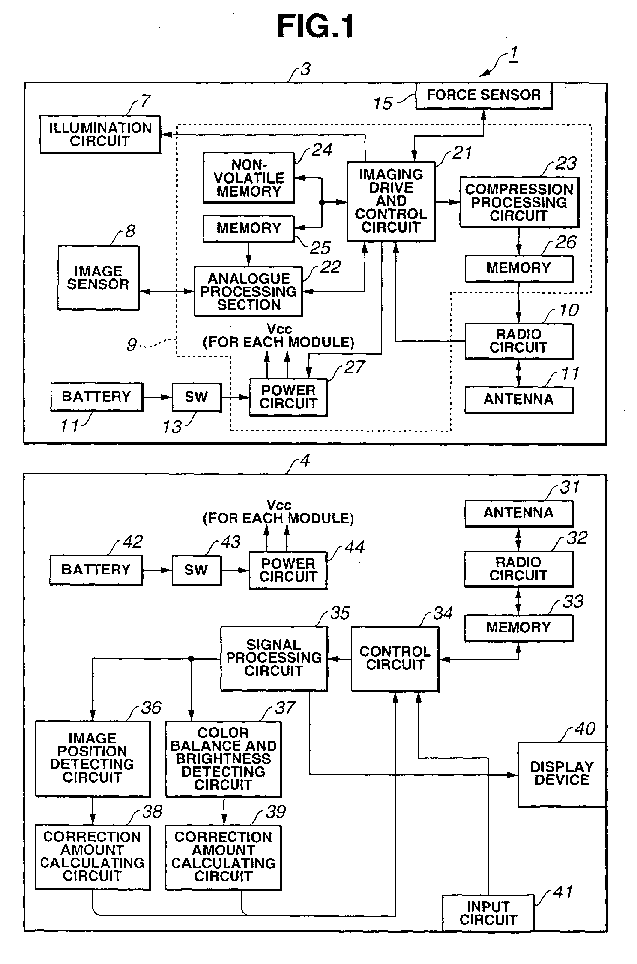

A first embodiment of the present invention is described with reference to Fig. 1 to Fig. 12B.

As shown in Fig. 1, a capsule medical device 1 according to the first embodiment of the present invention comprises: a capsule medical device main body (hereinafter, simply called the capsule) 3, which is capsule-shaped, incorporates an image sensor for capturing images therein, and which is inserted into a body cavity of a patient (not illustrated), by means of swallowing or the like; and an external device 4 disposed outside the body for receiving radio signals transmitted by radio waves from the capsule 3, setting the capsule 3 to more desirable medical actions and operational states in accordance with the received signals, and accumulating information obtained from the capsule 3.

In this capsule medical device 1, after pre-treatment of the colon (bowel irrigation), the capsule 3, which is shaped like a capsule, is swallowed together with water or the like, similarly to medicine, and a screening inspection of the oesophagus, duodenum, small intestine and large intestine can be performed.

Furthermore, the external device 4 receives image data transmitted by radio by the capsule 3, detects the operational state of the capsule 3, such as the image pick-up state thereof, transmits corrected data and the like, in order to establish a suitable operational state with respect to items that require adjustment after assembly and the like, and stores data in storing means of the capsule 3 (a non-volatile memory or a memory), whereby the operations of the capsule 3 can be set to a suitable operational state.

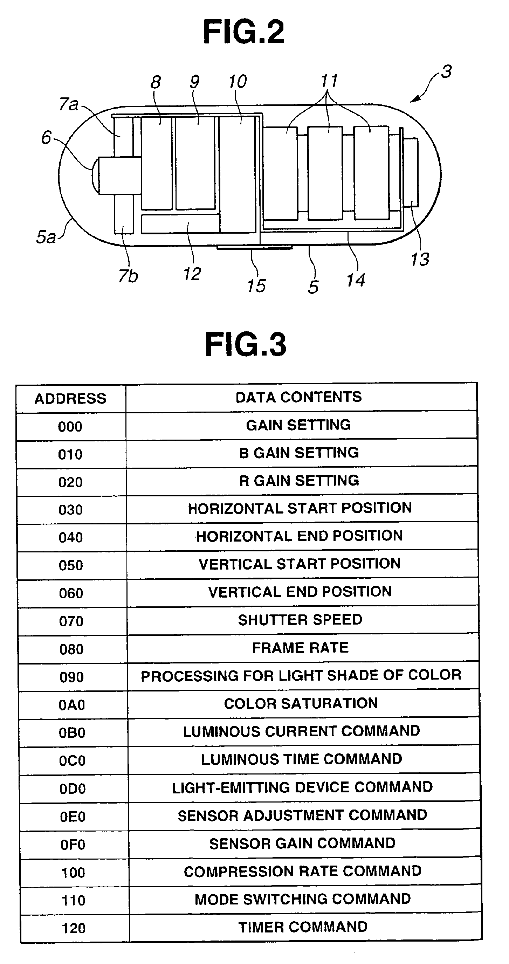

As shown in Fig. 2, in the capsule 3, one end section (front end) of a closed, tubular-shaped or capsule-shaped, accommodating vessel 5 is formed by a hemispherical transparent member 5a, an objective optical system 6 being disposed in a lens frame in a position opposing the transparent member 5a, in the vicinity of the center thereof, and a plurality of white LEDs 7a, 7b, for example, being disposed as light-emitting devices forming an illuminating circuit 7 (see Fig. 1), in four positions, for instance, surrounding the objective optical system 6.

An image sensor 8, such as a CMOS sensor or the like, is disposed at the image formation position of the objective optical system 6. A signal processing and control circuit 9 for processing signals relating to the image sensor 8 or the like, and providing overall control, a radio circuit 10 for performing radio communications, and a plurality of button type batteries 11 for supplying operating power to the image sensor 8, the signal processing and control circuit 9 and the like, are disposed to the rear side of the image sensor 8.

Moreover, an antenna 12, connected to the radio circuit 10, for transmitting and receiving radio waves and thus performing radio communications with the external device 4 is disposed in a side region adjacent to the image sensor 8, and a switch 13 for switching the electric power supply on and off is disposed adjacently to the battery 12. Moreover, the image sensor 8, signal processing circuit 9 and radio circuit 10 are electrically connected by means of a flexible substrate 14, and this flexible substrate 14 is connected to the battery 11 via the switch 13.

Furthermore, a force sensor 15 is installed in such a manner that the sensor portion thereof is exposed externally beyond the accommodating vessel 5, and signals detected by the force sensor 15 are input to the signal processing and control circuit 9, in such a manner that movement of the capsule 3 can be detected.

Fig. 1 shows the more detailed internal composition of the electrical system of the capsule 3 and the external device 4.

The illumination circuit 7 comprises the white light LEDs 7a, 7b shown as light-emitting devices in Fig. 2 and a light emission driving circuit, and the inner walls or the like, of the body cavity illuminated by the white light LEDs 7a, 7b are formed into an image by the objective optical system 6. The image pick-up surface of the image sensor 8 forming imaging means is positioned at the image formation position of this image, and hence an image is picked up by the image sensor 8.

The illumination circuit 7 is controlled by an imaging drive and control circuit 21 which forms part of the signal processing and control circuit 9. Moreover, the image sensor 8 is driven via an analogue processing section 22, and the image picked up signals are processed by the analogue processing section 22, and then converted to digital signals (image data) by the imaging drive and control circuit 21, whereupon they are input to a compression processing device 23 and compressed.

Moreover, the force sensor 15 is also connected to the imaging drive and control circuit 21, and is controlled by the imaging drive and control circuit 21, which is constituted by a CPU or the like, and which detects movements of the capsule 3 from the signals detected by the force sensor 15.

The imaging drive and control circuit 21 is connected to a non-volatile memory 24 constituted by an EEPROM or the like (which maintains data even after the power is switched off), forming an electrically rewriteable data storage means, and a volatile memory (or register) 25 constituted by a static RAM or the like, which can be rewritten electrically at high-speed (and which loses data when the power is switched off). The CPU of the imaging drive and control circuit 21 performs imaging operations and the like, by referring to the information (data) stored in the non-volatile memory 24 and the memory 25.

In this case, the non-volatile memory 24 stores data principally for determining (specifying) the operations of the capsule 3 in an initial state. Moreover, the volatile memory 25 which can be rewritten at high speed, stores data for determining operations other than those in the initial state or the like, for use after the initial state has been established.

Fig. 3 shows the data contents stored in the memory 25, which include parameters, control information and the like, for determining the functions, operations and the like, of the capsule 3, stored at prescribed addresses. The external device 4 can transmit commands to the imaging drive and control circuit 21 in the capsule 3, thereby controlling the operations of the capsule 3 by means of these commands, in addition to which it is also able to implement settings for changing the operational parameters of the capsule 3, by instructing a rewrite of parameter data contained in the memory 25.

As shown in Fig. 3, the information stored relating to the image sensor 8 comprises gain settings, and B and R gain settings; the information relating to the imaging range comprises the horizontal start and end positions, the vertical start and end positions, shutter speed, frame rate, light color shade processing for tone control, color saturation, luminous current relating to the illumination light quantity or exposure light quantity, luminous time, and light-emitting device instructions; the information relating to the operation of the force sensor 15 comprises sensor adjustment instructions, sensor gain instructions, and compression rate instructions relating to the image compression information; and the information relating to imaging (image capturing) comprises mode switching instructions, timer instructions, and other such data.

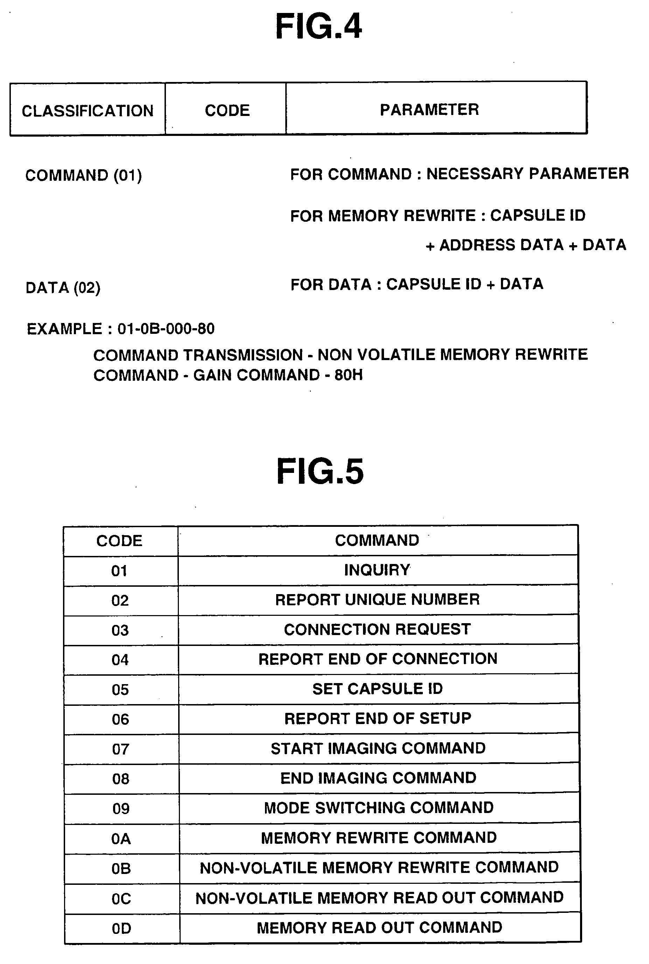

Fig. 4 shows a format used when transmitting commands or data from the external device 4 to the capsule 3.

As Fig. 4 shows, a format is adopted wherein an identification element which indicates a command (01) or data (02) is stated in a header, and then command and parameter elements are written subsequently. Here, in the case of a command as illustrated in Fig. 5, after a code (command code), the parameters required for that command are specified.

On the other hand, in the case of data transmission, after identifying the data, the capsule ID and data are stated.

Furthermore, in the case of a memory (register) rewrite operation, the parameters are written in the form: capsule ID + address + data.

Fig. 4 shows one concrete example.

More specifically, this shows a transmission example, "01 - 0B - 000 - 80". The information transmitted in this transmission is as follows.

Namely, "command transmission - non-volatile memory rewrite command - gain command - 80H"

In other words, the "01" element indicates that a command is being transmitted, and the instruction in that command is indicated by "0B", which indicates a rewrite of the non-volatile memory, the address of the rewrite is "000", which indicates the gain setting, and "80" indicates that the gain value is to be set (rewritten) to 80H.

The (CPU) of the imaging drive and control circuit 21 in the capsule 3 performs operations corresponding to the command or the like, transmitted by the external device 4 in the format shown in Fig. 4.

Moreover, Fig. 5 shows a concrete example of code - command (instruction) correspondence information stored in an internal ROM or the like, of the imaging drive and control circuit 21 in the capsule 3. When code information corresponding to a command as illustrated in Fig. 5 is transmitted by the external device 4, the command information corresponding to that code information is read out (extracted) and operations corresponding to that command are carried out.

Furthermore, virtually similar correspondence information to that shown in Fig. 5 is also stored in the external device 4, as described hereinafter, whereby command information corresponding to code information transmitted by the capsule 3 is read out and corresponding operations are performed.

If the capsule 3 principally transmits image picked up image data to the external device 4, then this is transmitted in the format shown in Fig. 4.

The image data compressed by the compression processing circuit 23 is stored in a memory 26, and the data is then read out from the memory 26, and transmitted to the radio circuit 10, which performs high-frequency modulation and emits the data as a radio wave from the antenna 11.

Moreover, the DC power supply from the battery 11 is supplied via the switch 13 to a power circuit 27, which converts the power into a suitable voltage for operating the respective modules (circuits), which is supplied to the power terminal Vcc. This power circuit 27 is also controlled by the imaging drive and control circuit 21.

For example, by setting a portion of the power circuit 27 to a rest state or the like, it is possible to shut off the electrical power supplied to a portion of the circuits inside the capsule 3, and hence the circuits which have been shut off will assume an idle state, thus preventing wasteful power consumption by those circuits.

On the other hand, the external device 4 receives the radio waves transmitted by the capsule 3 via an antenna 31, and after demodulating the signal in a radio circuit 32, it stores the data in a memory 33. The image data stored in the memory 33 is read out by a control circuit 34 and transmitted to a signal processing circuit 35, which performs expansion processing and the like, thereby regenerating the image data to its state before compression.

This image data is then supplied to an image position detecting circuit 36 and a color balance and brightness detecting circuit 37, which respectively detect the image position and the color balance and brightness.

The respective detection signals are sent to correction amount calculating circuits 38 and 39, which calculate the required correction amounts. The respective correction amounts calculated respectively by the correction amount calculating circuits 38 and 39 are sent to the control circuit 34, and the control circuit 34 then stores data for the correction amounts in the memory 33. The data stored in the memory 33 can be modulated at a high frequency by the radio circuit 32 and transmitted to the capsule 3 in the form of radio waves via the antenna 31.

The control circuit 34 is also constituted by a CPU or the like, and the command codes shown in Fig. 5 are stored in an internal ROM or the like, of the control circuit 34, in such a manner that when data relating to correction amounts is transmitted, a format is used wherein the data is added to a rewrite command or the like, relating to the memory 25.

Moreover, the signal processing circuit 35 or control circuit 34 are connected to a display device 40, which displays images obtained by expansion processing, as well as the capsule ID transmitted by the capsule 3 and the like, in such a manner that they can be viewed or monitored by the user.

The control circuit 34 is also connected to an input circuit 41 constituted by a keyboard or the like, and when controlling the operations or the like, of the capsule 3, from the external device 4, commands can be input via this input circuit 41.

Commands, data or the like, input from the input circuit 41 are stored in the memory 33 via the control circuit 34, and can then be transmitted in the form of radio waves, in a similar manner to the correction amount data or the like.

Furthermore, a battery 42, switch 43 and power circuit 44 are provided inside the external device 4, and the DC voltage generated by the power circuit 44 is supplied to the power terminal Vcc of each module (circuit).

The radio waves emitted by the antenna 31 of the external device 4 are received by the antenna 11 of the capsule 3 and are demodulated by the radio circuit 10, whereupon the signal is sent to the imaging drive and control circuit 21. The CPU forming this imaging drive and control circuit 21 stores the demodulated data in the memory 25 or the like, and the capsule 3 is caused to operate or the like, on the basis of the stored data.

In the present embodiment having a composition of this kind, the non-volatile memory 24 for storing information for initial setup operations, and the memory 25, which is volatile and is capable of controlling the operational state and the like, of the capsule 3 by information being written thereto, are provided in the capsule 3, and even after the capsule 3 has been assembled, the user, such as a medical practitioner or the like, is still able to adjust the capsule 3 to a suitable operational state, by changing the information written to the memory 25 or the like, in addition to which, he or she is also able to control the subsequent operational state, independently, in accordance with the information written to the memory 25.

A typical example of the operation of the present embodiment will now be described.

The power supply to the capsule 3 and the external device 4 is switched on, thereby setting both to an operational state. The CPU of the control circuit 34 of the external device 4, for example, then transmits an inquiry command of code 01 as illustrated in Fig. 5, to find out whether or not a capsule 3 is present in the vicinity. Upon receiving this command, the capsule 3 evaluates the command, and takes the capsule ID containing the peculiar number written into the capsule 3 upon manufacture, appends the capsule ID to a peculiar number notification command, and transmits same to the external device 4.

The external device 4 extracts (acquires) the capsule ID, and stores it in an internal register or the like, of the control circuit 34, whilst also displaying the capsule ID on the display device 40.

If only one capsule 3 is being used, then the medical practitioner or other type of user transmits a connection request command to the capsule 3 having the capsule ID thus obtained, via the input circuit 41 of the external device 4, whereupon, by receiving a connection completed notification command from the capsule 3, the capsule 3 and the external device 4 are set respectively to a state for performing bidirectional radio communications.

Thereupon, the operational state of the capsule 3 can be set by transmitting a command, such as an imaging start command or the like, to the capsule 3 via the input circuit 41. In this case, before the patient swallows the capsule 3, a white reference object, such as a white sheet or the like, is placed in front of the transparent member 5a of the capsule 3, and an imaging start command is transmitted.

Upon receiving this command, the capsule 3 evaluates the command, and consequently starts illumination by means of the illumination circuit 7 and imaging by means of the image sensor 8.

The non-volatile memory 24 stores control information for the imaging mode in the initial setting, such as single-image mode, for example, as described hereinafter, and the CPU of the imaging drive and control circuit 21 of the capsule 3 performs an imaging operation and the like, in accordance with the control contents written to the non-volatile memory 24 (in an initial state, the contents of the memory region of the memory 25 are cleared).

The image picked up image data is compressed and transmitted to the external device 4, which expands the transmitted image data by means of the signal processing circuit 35, and then outputs to the image position detecting circuit 36 and color balance and brightness detecting circuit 37.

In the image position detecting circuit 36, the luminance distribution of the image picked up image is calculated and output to the correction amount calculating circuit 38.

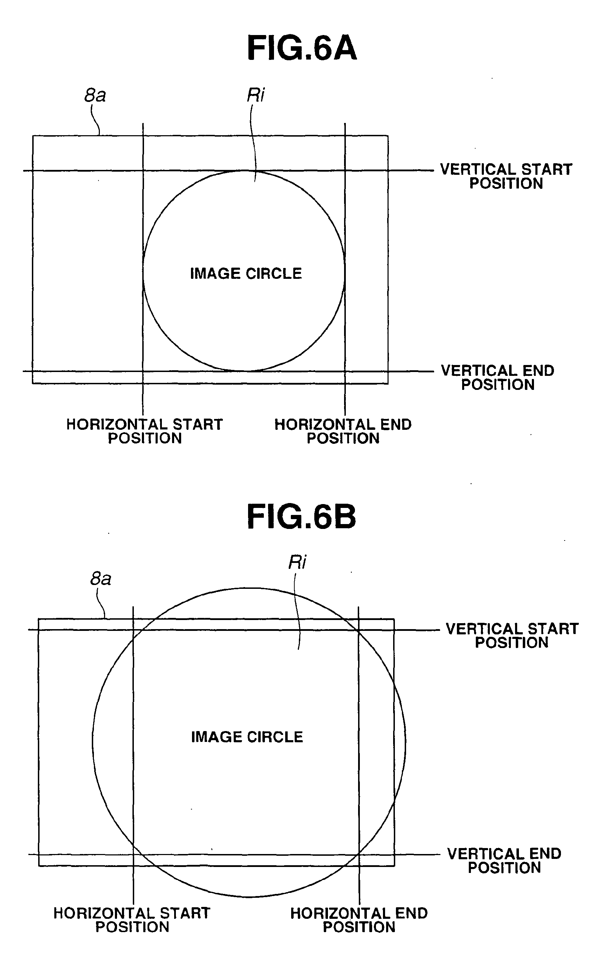

In the correction amount calculating circuit 38, depending on whether or not a predetermined threshold value has been exceeded or not, an image circle Ri forming the effective imaging range of the objective optical system 6 is calculated, as illustrated in Fig. 6A, and furthermore, a horizontal start position, horizontal end position, vertical start position and vertical end position on an image pick-up surface 8a of the image sensor 8 previously set in such a manner that they are defined by vertical lines and horizontal lines circumscribed to the calculated image circle Ri, for example, are calculated respectively and sent to the control circuit 34.

The control circuit 34 adds this positional data as correction amount data to a memory rewrite indicator command, as illustrated in Fig. 5, and transmits it to the capsule 3. The capsule 3 stores the positional data thus received in the memory regions of the memory 25 at addresses 30, 40, 50 and 60 shown in Fig. 3.

Thereafter, the capsule 3 transmits only the image data inside the square shaped imaging region determined by this positional data, to the external device 4. In this way, by setting (adjusting) the imaging region after the capsule 3 has been assembled, it is possible to make suitable adjustments in such a manner that data obtained in the pixel regions where no image is actually picked up is not transmitted to the external device 4. By making adjustment in this way, it is possible to simplify the adjustments required when assembling the objective optical system 6 and the image sensor 8 in the capsule 3.

In the case of Fig. 6A, the image range in which an image is formed by the objective optical system 6 lies inside the imaging range of the image sensor 8, but similar settings are also made in a case such as that shown in Fig. 6B, where the image range formed by the objective optical system 6 extends beyond the imaging range of the image sensor 8.

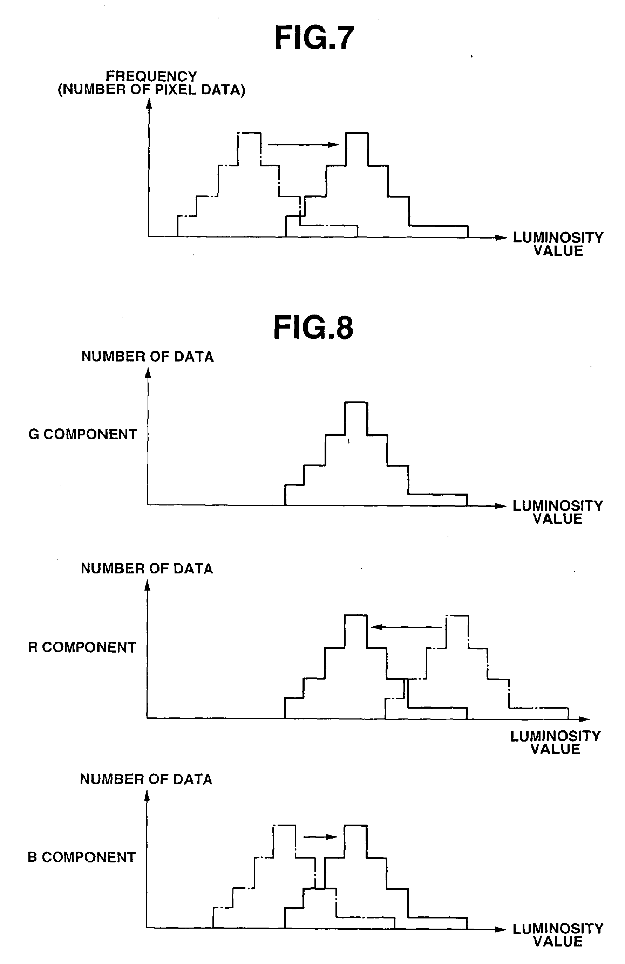

Furthermore, the color balance and brightness detecting circuit 37 detects (calculates) the histogram of the brightness (luminance) in the image picked up image, as in the portion of Fig. 7 marked by the single-dotted line, for example. The color balance and brightness detecting circuit 37 then transmits the histogram thus detected to the correction amount calculating circuit 39.

Reference data for a histogram of standard luminance distribution positions is previously stored in the correction amount calculating circuit 39, and correction amounts are calculated on the basis of this reference data, in such a manner that the luminance distribution indicated by the solid line in the diagram is achieved.

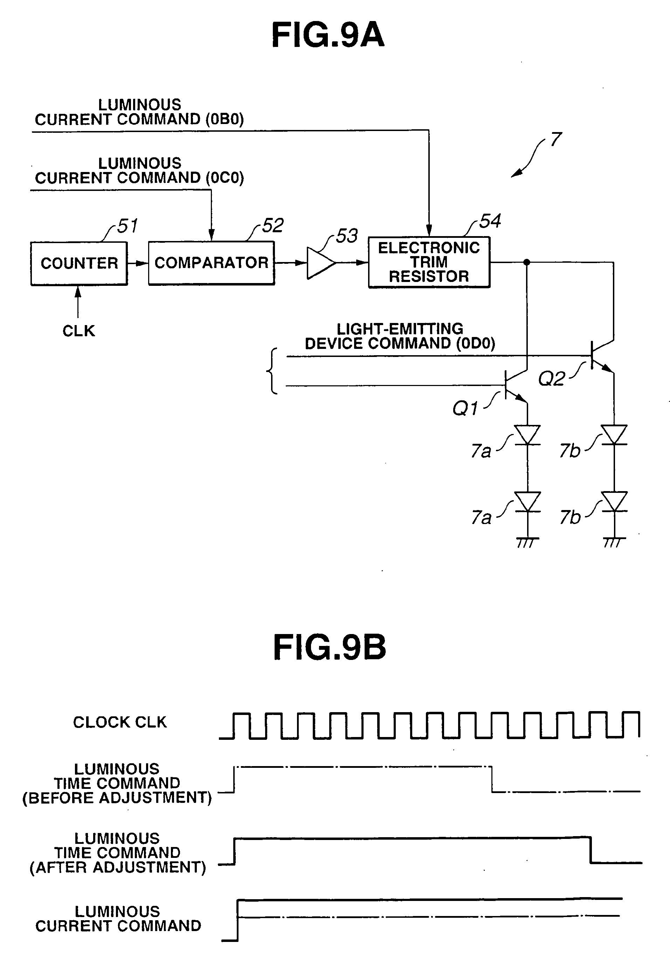

These correction amounts are transmitted by the control circuit 34 to the capsule 3, and luminous amount by the illumination circuit 7 of the capsule 3 is adjusted (as described hereinafter with respect to Fig. 9A and Fig. 9B), thereby achieving the histogram illustrated by the solid line.

Moreover, the color balance and brightness detecting circuit 37 calculates the luminance distribution of the green (G), blue (B) and red (R) components in the image picked up image, as illustrated in Fig. 8.

The luminance distribution for the G component is set substantially to a suitable state by adjusting the luminance distribution of the luminance in Fig. 7. On the other hand, the R and B components generally diverge from the suitable state indicated by the solid line, in such a manner that they require color balancing.

In the example in Fig. 8, for example, the R component is shifted to the high luminance side and the B component is shifted, conversely, to the low luminance side. In this case, correction amounts are calculated by the correction amount calculating circuit 39 and then sent to the control circuit 34.

These amounts are then transmitted by the control circuit 34 to the capsule 3, and the R gain setting and G gain setting data in the memory 25 of the capsule 3 are changed accordingly, in such a manner that they assume suitable states (from an initial state of zero). By adjusting these gain values, the luminance distribution is adjusted in such a manner that the histogram illustrated by the solid lines is achieved. In other words, the image signal is set to a color balanced (white balanced) imaging state wherein a color image signal which is displayed as white is generated when a white object is imaged.

The concrete brightness adjustment described above is carried out by controlling the illumination circuit 7 as illustrated in Fig. 9A, for example.

The illumination circuit 7 shown in Fig. 9A comprises two white light LEDs 7a connected in series between an the emitter and ground of a switching transistor Q1, and when the signal controlling the light-emitting devices applied to the base of the switching transistor Q1 is switched on, then light emission is driven by means of current flowing through the collector side. The two white light LEDs 7b are driven in a similar manner by a switching transistor Q2.

The collectors of the transistors Q1 and Q2 are devised so as to receive a luminous power supply via a counter 51, comparator 52, driver 53 and electronic trim resistance 54.

In this case, the clock CLK illustrated in Fig. 9B is input to the counter 51, and the output calculated by this counter 51 is input to the comparator 52, which compares this input with the signal value of the reference luminous time command.

During the time period of the reference luminous time command, an "H" signal is output to the driver 53, and a luminous current command signal is applied to an electronic trim resistor 54, whereby the resistance value of the electronic trim resistor 54 can be set to be varied.

For example, in the state of initial settings before adjustment, more specifically, in a case where the luminance distribution is as indicated by the single-dotted line in Fig. 7, if the value of the luminous time command is as shown by the single-dotted line in Fig. 9B, then by means of an adjustment command from the correction amount calculating circuit 39, the luminous time command after adjustment will be as shown by the solid line in Fig. 9B.

The signal value of the luminous time command is supplied to the comparator circuit 52, and the increased signal value is set.

By increasing (lengthening) the value of the luminous time command in this way, the luminous amount is increased, and hence the histogram of the luminance in the captured image will be set (adjusted) to that illustrated by the solid line in Fig. 7. In this case, it is also possible to set the histogram to the state illustrated by the solid line in Fig. 7 simply by lengthening the luminous time, but generally, a command for increasing the luminous current is also used conjointly.

In other words, if the luminous current command has the value illustrated by the single-dotted line in Fig. 9B in the initial settings, then it is increased so as to have the value illustrated by the solid line in Fig. 9B after adjustment.

Moreover, it is also possible to increase the luminous amount by changing from one light-emitting device command signal to two, or the like.

In this way, by increasing the luminous amount, it is possible to set the illumination and imaging state in such a manner that images having a suitable luminance distribution are obtained.

Fig. 9A shows the addresses corresponding to the data contents of the memory 25 in Fig. 3, for reference.

Furthermore, in the present embodiment, it is possible to adjust the gain and the like, of an adjustment circuit in such a manner that a stable operational state is achieved for the force sensor 15.

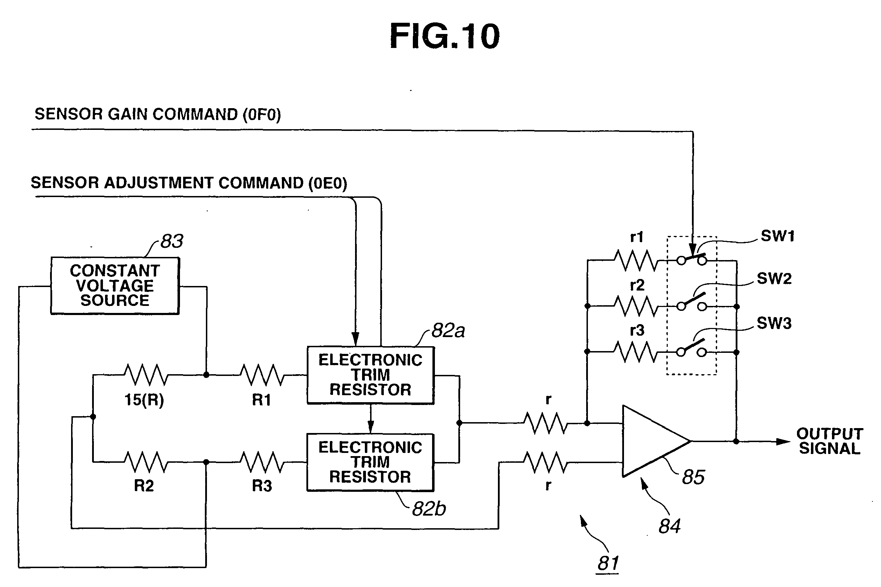

Fig. 10 shows an adjustment circuit 81 for the force sensor 15, and the adjustment circuit 81, excluding the force sensor 15 itself, is constituted by a portion of the imaging drive and control circuit 21, for example.

A bridge circuit is constituted by the force sensor 15 (the equivalent resistance thereof is illustrated as R), three resistances R1, R2, R3, and electronic trim resistors 82a, 82b connected respectively in series to the resistances R1 and R3, and a constant voltage is supplied to this circuit by a constant voltage source 83.

The resistance values of the electronic trimmers 82a, 82b can be set variably by means of a sensor adjustment command signal, in such a manner that the bridge circuit can be adjusted so as to assume a balanced state when no force is applied to the force sensor 15.

Moreover, from a state where no force is applied to the force sensor 15, if a force is then applied to the capsule 3, this will act on the force sensor 15, thus causing a slight change in the resistance value R thereof, whereby the balanced state of the bridge circuit is disrupted and a signal output having a small voltage is produced by the bridge circuit, amplified by a differential amplifier circuit 84, converted from analogue to digital and supplied to the imaging drive and control circuit 21.

In the differential amplifier circuit 84, signals passing through two resistances r connected to the bridge circuit are input to an operational amplifier 85 and the gain of this operational amplifier 85 can be set variably by turning on and off switches SW1, SW2, SW3 which are respectively connected in series to resistances r1, r2, r3. In this case, the gain is adjusted in accordance with the sensor gain command illustrated in Fig. 3.

By setting this gain to a suitable value, it is possible to ensure that the force or the like, acting on the capsule 3 can be detected in a stable and secure manner by the force sensor 15.

Consequently, according to the present embodiment, the color imaging function and the illumination function for imaging based on the capsule 3 can be set to suitable states after assembly of the capsule 3, and the sensor state of the force sensor 15 can be set to a suitable and stable operational state.

Moreover, once a state has been achieved where the region of the part inside a human body cavity that is to be investigated by means of the capsule 3 is being illuminated and imaged, then it is possible to change the operational state of the capsule 3, from an external device 4, for example.

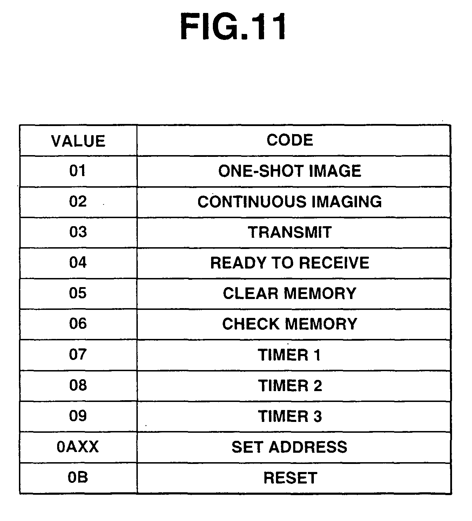

More specifically, commands for switching modes as illustrated in Fig. 3 can be supplied by operating the input circuit 41 of the external device 4, whereby the imaging mode can be changed from the single-frame imaging mode shown in Fig. 11 to a continuous imaging mode.

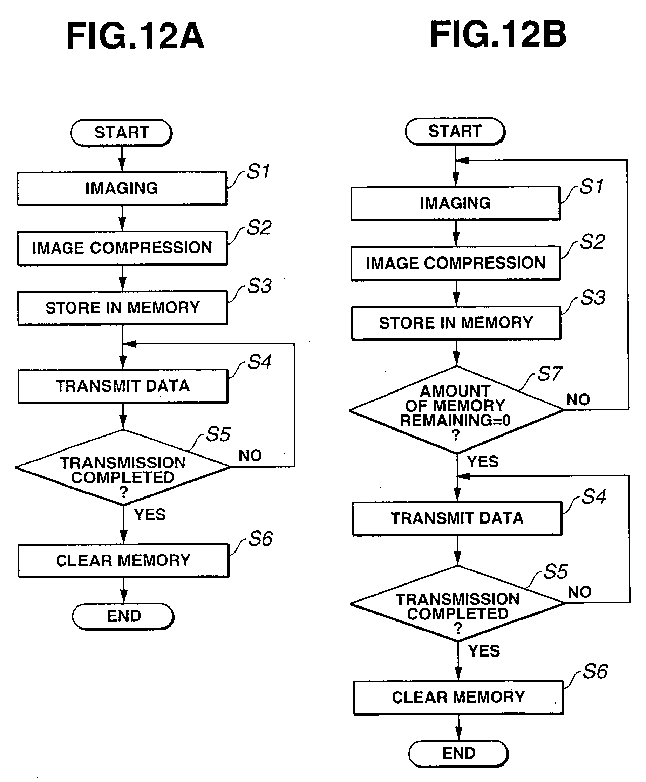

In the single-frame imaging mode, the imaging processing illustrated in Fig. 12A is carried out, and in the continuous imaging mode, the imaging processing illustrated in Fig. 12B is carried out.

In the single-frame imaging mode in Fig. 12A, when performing imaging by means of the image sensor 8 of the capsule 3, as indicated in step S1, the image thus captured is processed in analogue form by the analogue processing section 22, and is then converted to a digital signal, whereupon, as indicated in step S2, the signal is compressed (by the compression processing circuit 23).

As illustrated in step S3, the compressed image data is accumulated in the memory 26. The compressed image data accumulated in the memory 26 is then transmitted by radio waves, via the radio circuit 10 and the antenna 11, as indicated in step S4. Data transmission is carried out until the image data for one frame has been completed, as indicated in step S5, and when the image data for one frame has been transmitted, the sequence advances to step S6, where the memory 26 is cleared and the single-frame imaging operation ends.

Since this mode captures single-frame images in this way, it is suitable for use in confirming the operation of the capsule 3 after assembly, and in obtaining the required imaging information and the like, during the procedure for adjusting the color balance, for instance, without causing the capsule 3 to perform repeated illumination and imaging operations unnecessarily, thus avoiding wasteful power consumption.

On the other hand, in the continuous imaging mode in Fig. 12B, processing for determining whether or not the amount of memory remaining in the memory 26 is 0 is carried out between steps S3 and S4 in Fig. 12A, and if the amount of memory remaining in memory 26 has not become 0, then the sequence returns to step S1, and the processing from image capture in step S1 to step S4 is repeated.

In other words, image capture is repeated until the amount of memory remaining in the memory 26 becomes 0. By means of this step S7, after repeating image capture until the remaining memory in the memory 26 has become 0, the data transmission in step S4 is carried out and this transmission is performed until all data transmission has been completed in step S5 (in other words, until all of the plurality of image data captured until the remaining memory in the memory 26 became 0 have been transmitted), whereupon the memory 26 is cleared.

In this way, in the present embodiment, a single-frame imaging mode and a continuous imaging mode are prepared, in such a manner that imaging can be carried out in a mode suited to the use conditions.

Moreover, as shown in Fig. 11, the time interval at which continuous imaging or the like, is performed inside a body cavity, can be set to any one of three time intervals specified by timers 1 to 3, for example, which can be selected in accordance with the object under investigation or the like, thus improving ease of use.

Furthermore, as shown in Fig. 11, codes for other functions are provided, such as a code for performing data transmission, a code for reporting that the device has assumed a receivable state, a code for clearing the memory 26 or the like, a code for checking the memory 26 or the like, a code for setting an address in the memory 25 or 26, or a code for performing a reset, and the like, thus providing a wide range of functions which allow the operations and the like, of the capsule 3, to be controlled in a more detailed fashion.

Moreover, when capturing images inside a body cavity, it is also possible to adopt a composition wherein the brightness is controlled, by means of the amount of illumination light or the like, emitted during a suitable time interval (for example, the imaging interval of around several times).

In other words, the average brightness of a plurality of images captured at the aforementioned time intervals is detected by the external device 4 and compared with a reference brightness value for the suitable amount of illumination light in that state, whereby the external device 4 calculates correction amount data for correcting the brightness to the reference value and transmits the same to the capsule 3, in such a manner that the operation of adjusting the luminous amount by the white light LEDs 7a, 7b in the capsule 3 to a suitable value is performed continuously during imaging by the capsule 3.

By this means, in a state where the interior of a body cavity is being imaged by the capsule 3, when capturing images of a broad body passage section, such as the stomach or the like, the amount of illumination light can be increased in such a manner that images having a good S/N ratio are obtained, whereas conversely, when capturing images of a narrow body passage, such as the oesophagus or small intestine or the like, the amount of illumination light can be reduced in such a manner that images of a suitable brightness can be obtained. By obtaining images of a suitable brightness of this kind, it becomes easy to make a diagnosis.

Furthermore, in cases such as these, the amount of electrical energy from the battery 11 used for illumination can be adjusted suitably, and hence wasteful consumption of electrical energy can be prevented effectively.

Moreover, simple control can be performed in accordance with the area under observation, whereby imaging is performed at shorter time intervals in the vicinity of certain regions, whilst imaging is performed at long time intervals in regions distant from the region under observation, and hence the apparatus can be adapted readily to a wide range of applications.

Furthermore, by transmitting a command signal instructing that the information obtained by the force sensor 15 be transmitted at suitable intervals from the external device 4 side, the external device 4 is able to monitor the movements of the capsule 3 by means of the force sensor 15, and hence it becomes very straightforward to detect when the capsule 3 has stopped moving, by the signal from the force sensor 15, and hence rapid response also becomes possible.

Consequently, according to the present embodiment, it is possible to set the color imaging function and the illumination function for imaging to suitable states in the capsule 3 after assembly, and it is also possible to set the state of the force sensor 15 to a suitable and stable operational state.

Furthermore, even after adjustment, by changing the parameters or the like, of the memory 25 in the capsule 3, and issuing switching commands and the like, it is also possible to change the operational state of the capsule 3, appropriately, in accordance with the region under examination or the like. Therefore, ease of use can be improved to a large extent compared to the prior art.

Moreover, it is also possible to obtain image information and the like for the object under examination, in a suitable operational state and using suitable settings, according to the object under examination. More specifically, depending on the object under examination, it is possible to prevent the electrical energy of the battery 11 from being consumed by taking an excessive number of images, or using excessive illumination, at excessively short imaging time intervals, and moreover, image information captured at suitable time intervals and having a suitable color balance can be obtained, in addition to which, images which make diagnosis easier to perform can be obtained by means of an illumination state of suitable brightness.

Since the contents of the non-volatile memory 24 can be written after assembly has been completed, then the initial settings can be changed after assembly, by checking operation after assembly, and then rewriting the contents of the non-volatile memory 24 on the basis of the data thus obtained.

In this way, according to the present embodiment, even after the capsule 3 has been assembled, it is still possible readily to set the capsule 3 to a suitable setup, or to change it to a more suitable operational state, by changing the information stored in the storing means.

### (Second embodiment)

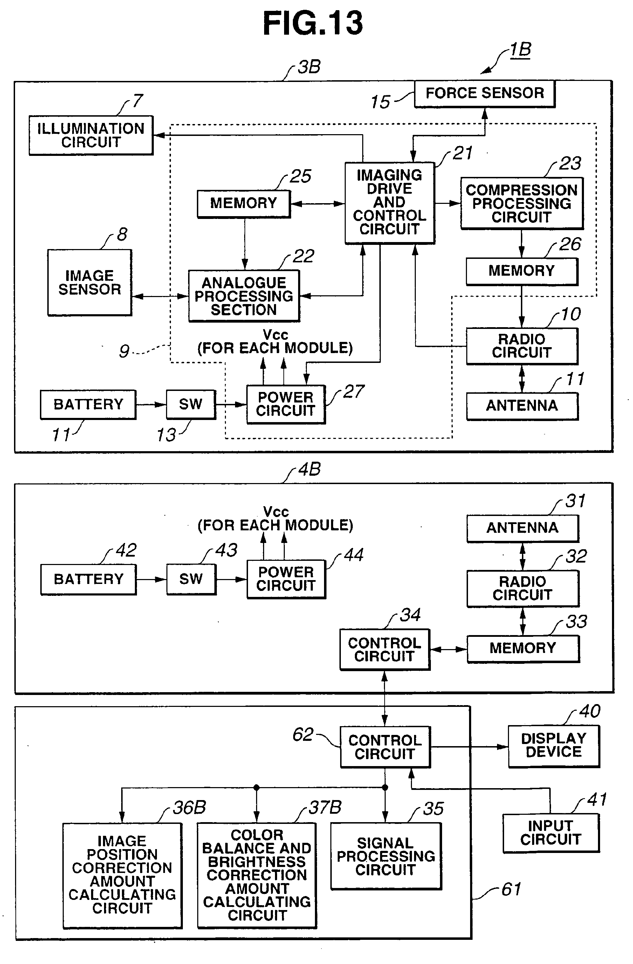

Next, a second embodiment of the present invention is described. Fig. 13 shows a capsule medical device 1B according to the second embodiment of the present invention.

The capsule medical device 1B shown in Fig. 13 comprises a capsule 3B, an external device 4B, and separately from this external device 4B, a display control device 61 having a display function, and the display device 40 and input circuit 41 connected to the display control device 61.

Compared to the capsule 3 in Fig. 1, the capsule 3B in Fig. 13 is not provided with the non-volatile memory 24, and only comprises the rewriteable, volatile memory 25, in such a manner that the information required for operating the capsule 3B is stored inside this memory 25 only.

By providing the functions of the memory 26 in this memory 25 also, it is possible to omit the memory 26.

Furthermore, compared to the external device 4 in Fig. 1, the external device 4B has a composition in which the functions of the image position detecting circuit 36 and the color balance and brightness detecting circuit 37 are moved to an external display control circuit 61.

Moreover, the display control circuit 61 has an in-built control circuit 62 for controlling display, and it transmits and receives data and the like, to and from the control circuit 34 of the external device 4B. This control circuit 62 is connected to an image position correction amount calculating circuit 36B and a color balance and brightness correction amount calculating circuit 37B which are respectively integrate the image position detecting circuit 36, the color balance and brightness detecting circuit 37 and the correction amount calculating circuits 38 and 39 illustrated in Fig. 1. Although not illustrated, the display control circuit 61 has a built-in battery, or a built-in power circuit for generating a fixed voltage supply from a mains AC power supply.

The remainder of the composition is basically the same as that of the first embodiment. In this embodiment, when the capsule 3B has been set to an operational state by switching on the power supply, the capsule 3 reads in data transmitted by the external device 4B and writes this data to the memory 25. Thereafter, virtually the same operations as those in the first embodiment, for example, can be performed, by means of the data written in the memory 25.

According to the present embodiment, it is possible to reduce the cost of the capsule 3B yet further. Apart from this, virtually the same beneficial effects as those of the first embodiment are obtained.

### (Third embodiment)

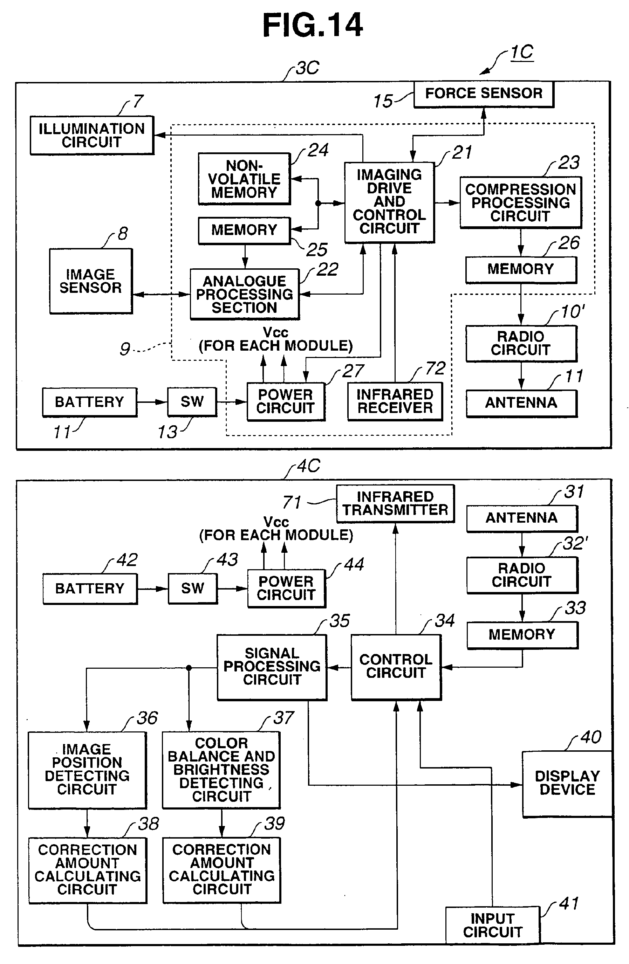

Fig. 14 shows a capsule medical device 1C according to a third embodiment of the present invention.

This capsule medical device 1C comprises a capsule 3C and an external device 4C, and compared to the capsule medical device 1 illustrated in Fig. 1, the capsule 3C of this capsule medical device 1C only performs data transmission to the external device 4C by means of radio waves, whilst the external device 4 is constituted in such a manner that it transmits data to the capsule 3C by means of infrared radiation.

Therefore, an infrared transmitter 71 connected to a control circuit 34 is provided in the external device 4C, and this infrared transmitter 71 transmits the data sent by the control circuit 34, by modulating it using an infrared beam.

The capsule 3C, on the other hand, is provided with an infrared receiver 72 connected to an imaging drive and control circuit 21, and this infrared receiver 72 receives the infrared beam sent by the infrared transmitter 71, demodulates it, and transmits the resulting signal to the imaging drive and control circuit 21.

A radio circuit 10' in the capsule 3C has a function for modulating the data from the memory 26 and transmitting it by means of a radio wave, from the antenna 11, but it does not have a function for demodulating radio waves.

A radio circuit 32' in the external device 4C has a function for demodulating radio waves received via the antenna 31 and outputting them to a memory 33, but it does not have a function for modulating radio waves.

According to this embodiment, it is possible to perform the same functions (operations) as in the first embodiment, after the capsule 3C has been assembled and before the capsule 3C has been swallowed by the patient.

Furthermore, when the patient swallows the capsule, it has generally been decided what kind of examination is to be carried out, and therefore the information required for that type of examination should be transmitted previously by means of an infrared beam, from the external device 4C to the capsule 3C, and stored in the memory 25.

According to the present embodiment, since data is transmitted by radio waves in one direction only, the composition of the radio circuit 10' and the 32' is simplified. Apart from this, virtually the same beneficial effects as those of the first embodiment can be obtained.

### (Fourth embodiment)

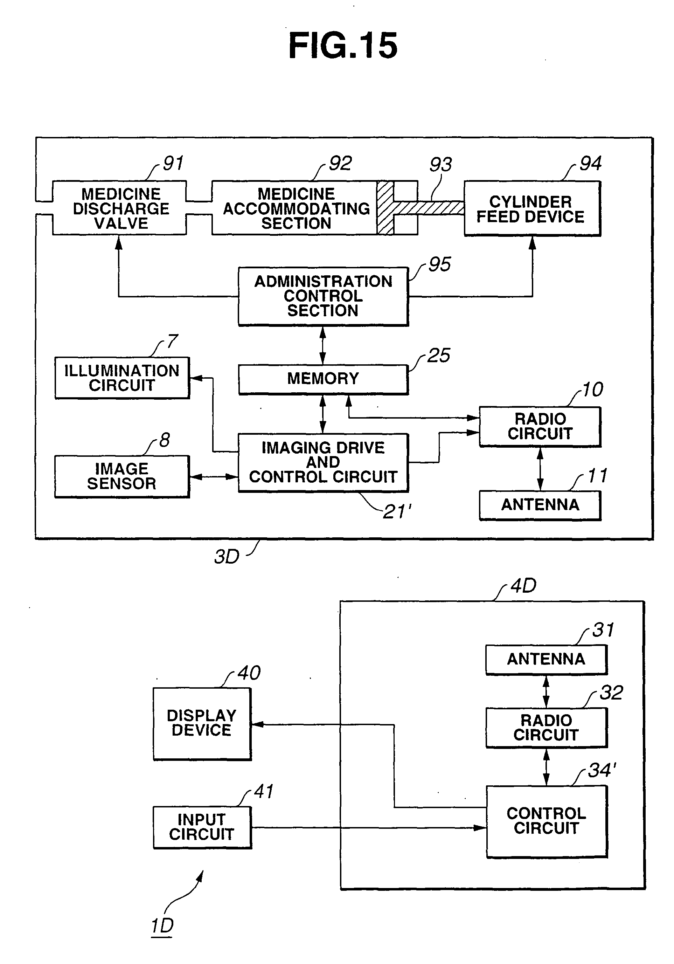

Fig. 15 shows a capsule medical device 1D according to a fourth embodiment of the present invention.

In addition to a function for capturing images (and providing illumination for imaging), as in the first embodiment, this capsule medical device 1D is also able to perform administration of medicine, whilst the images provided by the imaging function are verified.

For this purpose, the capsule 3D comprises an illumination circuit 7 and image sensor 8, an imaging drive and control circuit 21' for controlling these elements, a radio circuit 10 and antenna 11, and a memory (register) 25 for storing parameters, and the like, for determining the operation of the capsule 3D. The imaging drive and control circuit 21' is composed in such a manner that it also comprises the functions of the analogue processing section 22 illustrated in Fig. 1.

Moreover, in the present embodiment, the capsule 3D further comprises: a medicine discharge valve 91, opened in the outer surface of the accommodating vessel of the capsule 3D and capable of discharging medicine; a cylindrically-shaped medicine accommodating section 92, connected to the medicine discharge valve 91 by means of a tube, for accommodating medicine; a cylinder feed device 94 for driving a cylinder 93 which moves slidably in the medicine accommodating section 92; and an administration control section 95 for controlling the drive of the cylinder feed device 94 and controlling the opening and closing of the medicine discharge valve 91.

By transmitting parameters, and the like, relating to the imaging function from the external device 4D, similarly to the first embodiment or second embodiment, these parameters, and other such information, can be stored (recorded) in the memory 25, in addition to which commands, data and the like, for determining the control operations of the administration control section 95 can also be written to the memory 25.

The administration of the medicine can be performed by means of the administration control section 95, by transmitting a command from the external device 4 or the like.

Moreover, as described in the first embodiment, the external device 4D comprises an antenna 31 for transmitting and receiving radio waves, to and from the antenna 11 of the capsule 3D, a radio circuit 32 for performing modulation and demodulation, a control circuit 34', connected to the radio circuit 32, for performing signal processing and control with respect to the image data transmitted by the capsule 3D, a display device 40, connected to the control circuit 34', for displaying images and the like, and an input circuit 41 for performing input and the like, of commands and data for transmission to the capsule 3D.

The control circuit 34' depicted comprises the functions of the signal processing circuit 35 and image position detecting circuit 36 shown in Fig. 1, and the respective correction amount detecting circuits and the like. Moreover, in Fig. 15, the battery inside the capsule 3D and the battery in the external device 4D are omitted from the illustration.

The functions and operations of the present embodiment having a composition of this kind are now described in more detail.

An image sensor 8 constituted by a CCD, CMOS sensor or the like, is incorporated into the capsule 3D, which is swallowed by the patient, and an illumination circuit constituted by an LED or the like, for illuminating the interior of a body part of which images are captured by the image sensor 8 is provided adjacently to the image sensor 8.

The illumination circuit 7 and the image sensor 8 are connected to the imaging drive and control circuit 21', and this imaging drive and control circuit 21' controls the capture of images, and the transmission of images to the radio circuit 10 and the like.

Moreover, an antenna 11 for transmitting and receiving data is provided in the capsule 3D, and in addition to receiving radio signals transmitted by the external device 4D, image data is transmitted by means of radio waves to the external device 4D.

Furthermore, the antenna 11 is connected to the radio circuit 10, which demodulates signals received by the antenna 11. It also modulates signals that are to be transmitted, and these signals are then transmitted by means of radio waves via the antenna 11.

The radio circuit 10 is also connected to a memory (register) 25, in such a manner that information relating to the discharge of medicine, and information relating to the capture of images can be stored in the memory 25.

Furthermore, a cylindrical medicine accommodating section 92 for accommodating medicine is provided in the capsule 3D, and by driving a slidable cylinder by means of a cylinder feed device 94, it is possible for medicine to be discharged via the medicine discharge valve 91 to the exterior of the capsule 3D.

The medicine discharge valve 91 and the cylinder feed device 94 are controlled by the administration control section 95.

On the other hand, the external device 4D which transmits and receives signals to and from the capsule 3D, by radio waves, internally comprises a transmission and reception antenna 31, a radio circuit 32, and a control circuit 34'.

Furthermore, the control circuit 34' is connected to a display device 40, and images of the interior of a human body captured by the capsule 3D are displayed on the display device 40.

Moreover, the control circuit 34' is connected to an input circuit 41 whereby data for transmission to the capsule 3D is input. Data input in this manner is encoded by the control circuit 34', and transmitted as radio waves via the radio circuit 32 and the antenna 31, whilst on the capsule 3D side, it is demodulated via the antenna 11 and the radio circuit 10, and data is stored in, or rewritten to, the memory 25, in accordance with the encoded information.

The imaging drive and control circuit 21' checks the information held in the memory 25 constantly, and if an imaging request is written to the memory 25, then it executes imaging and transmits the captured images to the external device 4D by means of radio waves.

The external device 4D displays the images on the display device 40. Consequently, an operator (not illustrated) is able to obtain the images captured by the capsule 3D, at any time. Moreover, the imaging drive and control circuit 21' repeats the image capturing operation until a code for halting imaging is input to the memory 25.

The operator (not illustrated) is able to perform an operation for starting administration of the medicine, whilst confirming the images captured by the capsule 3D.

The operator inputs a command, via the input circuit 41, for opening the medicine discharge value 91. The input data is transmitted to the capsule 3D, by means of the external device 4D, and is stored in the memory 25.

The administration control section 95 constantly monitors the information in the memory 25, and when information for opening the medicine discharge value 91 is written to the memory 25, it causes the medicine discharge value 91 to open. The administration control section 95 maintains the medicine discharge value 91 in an opened state, until information for closing the medicine discharge value 91 is written to the memory 25.

Thereupon, the operator inputs data indicating a feed rate for the cylinder feed device 94 via the input circuit 41. The data thus input is transmitted to the capsule 3D by means of the external device 4D, and is stored in the memory 25.

The administration control section 95 constantly monitors the information in the memory 25, and when information on the feed rate for the cylinder feed device 94 is written to the memory 25, then the cylinder feed device 94 is caused to operate at a speed based on the data written to the memory 25. Thereby, the medicine (not illustrated) is pushed outside of the capsule 3D, via the medicine discharge value 91. Since the feed rate of the cylinder feed device 94 is specified, it is possible to control the amount of medicine discharge per unit time. The administration control section 95 maintains the feed rate of the cylinder feed device 94 until new feed rate information for the cylinder feed device 94 is written to the memory 25.

Accordingly, the operator is able to control the amount of medicine discharged in such a manner that, if the operator wishes to reduce the amount of medicine discharged, then he or she should enter data for slowing the feed rate of the cylinder feed device 94, whereas if the operator wishes to increase the amount of medicine discharged, then he or she should enter data for increasing the feed rate of the cylinder feed device 94.

According to the present embodiment, since it is possible to control the capsule 3D simply by writing prescribed data to the memory 25, and since it is possible to maintain continuity in operations by holding this information in the capsule 3D, then communications between the capsule 3D and the external device 4D can be simplified.

Moreover, if continuous imaging, continuous administration of medicine or the like, is to be implemented, then since information is stored in the capsule 3D, it is possible to perform continuous operations in a straightforward manner.

Furthermore, by previously accommodating no medicine in the medicine accommodating section 92 according to the present embodiment, and operating the cylinder feed device 94 in a reverse direction, then it is possible to take a sample of body fluid. In this case also, a similar control method is employed.

According to the present embodiment, when administering medicine, it is possible to confirm the location to which the medicine is to be administered, by observing images. In addition to this, virtually the same beneficial effects as those of the first embodiment are obtained.

Modifications of the embodiments constituted by incorporating parts of the various embodiments described above, or the like, are also included in the scope of the present invention.

### Industrial Applicability

As described above, in the capsule medical device according to the present invention, it is possible to change the settings and the operation of a sensor, or the like, incorporated into the device, and hence the device can be changed readily to more suitable settings and a more suitable operating state, thereby making it applicable to a wide range of uses.

## Claims

1. A capsule medical device inserted into a body cavity, comprising:
a receiving device for receiving data from outside the capsule medical device; and
a storage device wherein storage data stored therein can be rewritten on the basis of the data received by the receiving device.

2. The capsule medical device according to claim 1, wherein the storage device is a storage device in which the storage data is not erased, even when the power supply is switched off.

3. The capsule medical device according to claim 1, wherein the capsule medical device has a sensor; and
the sensor is operated based on information stored in the storage device.

4. The capsule medical device according to claim 1, wherein the capsule medical device has an image acquiring device; and
the image acquiring device is operated based on information stored in the storage device.

5. The capsule medical device according to claim 4, wherein the image acquiring device has an illumination device; and
the illumination device is operated based on information stored in the storage device.

6. The capsule medical device according to claim 4, wherein the image acquiring device has an image sensor; and
the image sensor is operated based on information stored in the storage device.

7. The capsule medical device according to claim 4, wherein the image acquiring device has an image data compressing device; and
the image data compressing device is operated based on information stored in the storage device.

8. The capsule medical device according to claim 1, wherein the capsule medical device has a force acquiring device; and
the force acquiring device is operated based on information stored in the storage device.

9. The capsule medical device according to claim 1, wherein the capsule medical device has a medicine discharging device; and
the medicine discharging device is operated based on information stored in the storage device.

10. The capsule medical device according to claim 1, wherein the capsule medical device has a specimen recovery device; and
the specimen recovery device is operated based on information stored in the storage device.

11. A capsule medical device system comprising:
a capsule medical device inserted into a body cavity;
an external device for transmitting and receiving data, to and from the capsule medical device, by means of radio communications; and
a storage device, provided in the capsule medical device, the storage contents of which are rewritten on the basis of information transmitted to the capsule medical device from the external device.

12. The capsule medical system according to claim 11, wherein the capsule medical device has a sensor; and
the sensor is operated based on information stored in the storage device.

13. The capsule medical system according to claim 11, wherein the capsule medical device has an image acquiring device; and
the image acquiring device is operated based on information stored in the storage device.

14. The capsule medical system according to claim 13, wherein the image acquiring device has an illumination device; and
the illumination device is operated based on information stored in the storage device.

15. The capsule medical system according to claim 13, wherein the image acquiring device has an image sensor; and
the image sensor is operated based on information stored in the storage device.

16. The capsule medical system according to claim 13, wherein the image acquiring device has an image data compressing device; and
the image data compressing device is operated based on information stored in the storage device.

17. The capsule medical system according to claim 11, wherein the capsule medical device has a force acquiring device; and
the force acquiring device is operated based on information stored in the storage device.

18. The capsule medical system according to claim 11, wherein the capsule medical device has a medicine discharging device; and
the medicine discharging device is operated based on information stored in the storage device.

19. The capsule medical system according to claim 11, wherein the capsule medical device has a specimen recovery device; and
the specimen recovery device is operated based on information stored in the storage device.

20. The capsule medical system according to claim 11, having a calculating device for generating transmission information that is transmitted from the external device to the capsule medical device, on the basis of data transmitted by the capsule medical device and received by the external device.

21. A data storing method for a capsule medical device, comprising the steps of:
transmitting data to a capsule medical device; and
storing the data in a storage device provided in the capsule medical device.

22. The data storing method for a capsule medical device according to claim 21, comprising the steps of:
confirming the operation of the capsule medical device;
creating corrected data on the basis of the results of operation;
transmitting the data to the capsule medical device; and
storing the data in a storage device provided in the capsule medical device.

23. A data storing method in a capsule medical device system comprising a capsule medical device and an external device for transmitting and receiving data, to and from the capsule medical device by means of radio communications, the method, comprising the steps of:
transmitting data to the capsule medical device from the external device;
receiving the data in the capsule medical device; and
storing the data in a storage device provided in the capsule medical device.

24. An operation modifying method in a capsule medical system comprising: a capsule medical device and an external device for transmitting and receiving data, to and from the capsule medical device by means of radio communications, the method comprising the steps of:
transmitting data from the capsule medical device;
receiving the data in the external device;
determining modified data on the basis of the data;
transmitting the modified data from the external device; and
storing the modified data in a storage device provided in the capsule medical device.
